# EUROPEAN PATENT APPLICATION

(11) **EP 3 121 321 A1**
(43) Date of publication of application: **25.01.2017**
(21) Application number: 15722249.8
(22) Date of filing: 18.03.2015
(51) Int. Cl.: D04B 1/18, A61F 13/08, A61F 13/10, D04B 1/24, D04B 1/26

(54) **SLEEVE AND DEVICE WITH GRADUATED COMPRESSION FOR THE TREATMENT AND/OR PREVENTION OF LYMPHOEDEMAS**

(30) Priority: 18.03.2014 PT 10753014
(71) Applicant: Barcelcom Têxteis S.A., 4754-909 Barcelos (PT)
(72) Inventor: DUARTE SOUSA COUTINHO, Gaspar Augusto, P-4150-387 Porto (PT); ESTEVES SOUSA FANGUEIRO, Raul Manuel, P-4700-150 Braga (PT); CORREIA DA CRUZ, Juliana Patrícia, P-4770-868 Castelões - V. N. Famalicão (PT); DE LACERDA LIMA, Clécio José, P-4800-040 Guimarães (PT); RIBEIRO PEREIRA, Albina Manuela, P-4710-374 Braga (PT)
(74) Representative: Patentree, Lda.
(86) International application number: PCT/IB2015/052002
(87) International publication number: WO 2015/140744

(57) **Abstract**

The present invention relates to a therapeutic sleeve made of knitted fabric with graduated compression that has multifunctional characteristics and that favours conditions for preventing the appearance or worsening of lymphoedema in limbs, avoiding swelling in limbs, and increasing the wearer's thermophysiological, ergonomic and psychological comfort during use.

This sleeve or therapeutic device, produced by small-diameter double cylinder knitting machines, contains structural modifications that enable several levels of compression to be obtained in the same tube of knitted fabric, with greater compression in the wrist region and less compression in the shoulder region.

The variation in compression, together with the type of fibrous materials used and the sleeve design, as well as the possibility of attaching it to a piece of clothing, help maximise the wearer's thermophysiological and psychological comfort during use.

## Description

### Technical domain

The present invention consists of a therapeutic device for treating and/or preventing lymphoedema and the relevant method for obtaining said device. Specifically, it consists of a therapeutic, multifunctional sleeve that has different levels of pressure along its length, to be used on upper or lower limbs to prevent lymphoedema. The present invention can also be used with a fibre structure that enables it to be more securely fastened to the body or to a regular garment and improves reduction of the lymphoedema.

### State of the art

Treatments for breast cancer, such as chemotherapy and radiotherapy, linked to the removal of mammary lymph nodes may cause the loss or inability to satisfy basic needs and integrity, thereby creating discomfort and suffering in patients. Problems with lymphoedema in upper limbs are one of the side effects of those treatments. This problem is a pathological situation characterised by the presence of a functional overload of the lymphatic system, in which the volume of lymph exceeds the amount carried by capillaries and vessels. These oedemas are difficult to treat, and treatment relies on the multidisciplinary approach prescribed by physicians, since the process of inflammation in the system causes complications and after-effects for lymphodynamics. Lymphoedema is an abnormal swelling of the upper limbs (arm, forearm and/or hand), which occurs after treatment or surgery for breast cancer. Lymphoedema occurs when the lymphatic vessels in the arm are unable to make the lymph flow. One of the greatest concerns related to this phenomenon is that the affected region is much more exposed to the occurrence of bacteria or infections. This phenomenon may occur months or years after surgery or treatment related to breast cancer.

Lymphoedema compromises the functional capacity and quality of life of those who suffer from it, because it causes muscle weakness that leads to reduced abduction, flexion and lateral rotation movements of the shoulder. It has been demonstrated that the appearance of lymphoedema causes several physical, psychological and social alterations that have a negative impact on the quality of life of those who have the condition.

Currently, the methods for treating the condition always include follow-up by a specialised therapist:
- skincare, encouraging hydration and cleansing;
- use of compression devices;
- physical exercises for the affected limb;
- lymphatic drainage to stimulate drainage of the lymph;
- use of medication to contain oedema.

The compression devices based on fibre-based systems, for this application, should be guided by the Medical Compression Armsleeves (RAL-FZ 387/2) standard, which contains the medical compression specifications for compression sleeves for upper limbs, following the classes shown in Table 1 for the wrist region. However, the compression intensity should be reduced in the wrist-shoulder direction; the compression between the wrist and the elbow should be reduced by 0-40% and the compression between the elbow and shoulder should be reduced by 10-60%. The reduction in compression is given in relation to the compression intensity at the wrist.

**Table 1: Compression classes**

| Class | Intensity | kPa | mmHg |
|---|---|---|---|
| 1 | Mild | 2.0-2.8 | 15-21 |
| 2 | Moderate | 3.1-4.3 | 23-32 |
| 3 | Strong | 4.5-6.1 | 34-46 |

As regards the use of medical compression devices to encourage lymphatic drainage in the day-to-day life of those who have the condition, it is necessary to maintain the standardised compression/decompression properties throughout the sleeve and introduce new functions, in order to promote an increase in functional capacity and quality of life for the wearer, by increasing flexibility, elasticity and permeability, reducing friction between the device and the skin, and increasing the thermophysiological control thereof. Furthermore, it should also give wearers the chance to attach this device to any piece of clothing and conceal the sleeve using the clothes that they normally wear.

Structures of knitted fabric based on seamless knitting machines have been developed for use in body-shaping supports, as described in document US7395557B1. The patent describes a garment made using seamless technology, with the aim of correcting posture without leaving lines on the wearer's back or bulges on the wearer's torso. The correction is achieved by introducing elasticated bands or straps in the area below the bust and in the dorsal and vertical ventral panels. These bands are incorporated into the structure of the knitted fabric. To achieve the desired effect, 95% polyamide fibres in microfibre are used, along with 5% elastane fibres.

Patent document US4367748A describes a girdle comprising two bodies: the outer body is composed of fabric that is purely for aesthetic purposes and the inner body is a combination of a ribbed structure and a latex structure. The product was developed to encourage anatomical correction of the wearer, massaging from the ribbed structure, while maintaining flexibility and skin hydration.

Patent document WO03014459 refers to a pair of tights with cosmetological, anti-ageing and antibacterial properties. The structure has the ability to gradually release active substances and control bacterial flora. The active substances are incorporated into the product by impregnating the garment after it has been made.

Document US0100024A1 refers to an anti-slip inner surface of elastic socks or sleeves to avoid them sliding against the surface of the skin.

Document US0154163A1 refers to a multiple sleeve method and apparatus for treating oedema and other swelling problems. The sleeve applies pressure to a part of the body and comprises: a first sleeve, which comprises a multiplicity of pressure-applying resilient protrusions configured to apply a therapeutic pressure to a surface of the body part of a patient, and a second sleeve that slides over the first sleeve that applies pressure to the first sleeve, thereby increasing the therapeutic pressure applied by the first sleeve on the limb.

Document US6254554B1 refers to a compression sleeve for lymphoedema problems. This sleeve is composed of an elastic fabric on the outside and is to be worn over the entire arm. It has straps to tighten the sleeve and provide different levels of compression along the arm.

These documents illustrate the technical problem to be solved by the present invention.

### General description

The present disclosure consists of a garment, specifically a circular knitted fabric sleeve, which is divided into two parts: the first part is the therapeutic sleeve itself, which starts at the wrist and extends to the armpit, the second part is a fibrous substrate that is responsible for extending the sleeve from the armpit region to the shoulder region, where it has several forms of fastening which will later be attached to the sleeve. The first part of the sleeve has the property of having graduated compression along the garment and functional elements that encourage the desired effect to enable a surprising reduction in oedema. The second part of the sleeve is responsible for fastening the entire system to the body or clothing in a much more efficient way.

This invention, unlike the documents mentioned above, includes multiple functions in a single garment. The knitted fabric sleeve is the result of a combination of at least two yarns: one responsible for the compression effect, elastomers double covered with polyamide, polyester or similar material, and another responsible for activating the functions incorporated in the raw material, based on functionalised polyamide, polyester or similar material. This product is formed of two types of knitted fabric, manufactured in a single tube, one based on rib knitted fabric and its derivatives, in the wrist-armpit region and another in a jersey knitted fabric and its derivatives between the armpit and the area where the device is fastened. These structures enable a greater functional capacity and quality of life for the wearer by using knitted fabrics that have graduated compression properties to assist the flow of lymph in the lymphatic system by releasing active substances that encourage hydration and cell regeneration of sensitive skin as well as being able to boost transcutaneous oxygenation of the skin to facilitate lymph flow. The selection of materials that favour breathability, moisture management, temperature regulation and bacterial control can also be an option in certain areas of the final product. Furthermore, ergonomic modification of the fastening of the sleeve to a piece of clothing or common bra or even the joining of two sleeves by incorporating several elements in the system will help boost the use of this product by medical prescription.

Therefore, in order to reduce oedema in the arm, a knitted fabric sleeve was developed that makes it possible to apply graduated compression along the arm in order to encourage lymphatic drainage of the upper limb and that enables improved ergonomics, thermophysiological and psychological comfort without compromising the indispensable therapeutic compression. This sleeve can be classified according to standardised compression classes (Table 1). This classification is performed having in mind the sleeve's compression performance for a span of perimeters in which it can be used, in order to ensure that the compression class applied is the one needed by the user even if his/her lymphoedema changes slightly during use.

It was found that by manipulating the fibrous materials and the architecture thereof a graduated compression sleeve is obtained that provides greater freedom of movement, greater thermophysiological and psychological comfort, as well as reducing areas of pressure on the skin, which is accentuated by removing the seams. The fibrous materials and their structural layout are the key to the excellence of the properties mentioned in this product and enable an astonishing reduction in oedema.

The therapeutic device proposed consists of a sleeve that covers the entire upper arm/forearm, particularly from the palm of the hand to the shoulder, and can include the entire limb or only some parts thereof (with or without the hand and/or with or without the shoulder). The different compression intensities applied by the sleeve are strategically located in the device to boost the therapeutic effect of lymphatic drainage and increase the limb's mobility while it is in use, thereby increasing its functional capacity as presented above. In terms of therapy, compression is graduated from the wrist to the armpit region in order to encourage lymphatic drainage in that direction. This aims to direct the flow of lymph using external compression on the skin and to increase mobility of the limb to trigger numerous forces on the lymphatic capillaries and ducts that will generate subcutaneous compression, created by body movement and, consequently, muscle movement.

This application describes a seamless circular knitted fabric sleeve for treating lymphoedema in one of the wearer's limbs, produced by small-diameter double cylinder knitting,
in which the sleeve comprises a range of areas with different compression levels in order to gradually reduce the compression applied to the wearer's limb in a distal to proximal direction,
in which the loop length of the knitted fabric and the yarn input tension are distributed along the sleeve so that compression varies between 15 and 46 mmHg in the first area of the sleeve, falling 0-40% up to a second area of the sleeve; and so that compression varies from the second area of the sleeve, falling 10-60% up to a third area of the sleeve.

In one embodiment, the sleeve of the present invention has a yarn tension that varies between 0.1 and 0.5 g/tex and the loop length varies between 0.002 and 0.08 mm, while the loop length in one area of the sleeve varies 5-20% in relation to the loop length in the preceding area.

In one embodiment, the knitted fabric of the sleeve of the present invention comprises at least two yarns: a first yarn and a second yarn.

In one embodiment, the knitted fabric of the sleeve of the present invention comprises a first yarn that is elastomeric with a linear mass density of between 200 and 300 dtex, which is double covered with man-made synthetic fibres that have a linear mass density of between 44 and 120 dtex and in which the man-made synthetic fibres are selected from a list composed of polyamides, polyesters, and blends thereof.

In one embodiment, the knitted fabric of the sleeve of the present invention comprises a second yarn that is made of a material selected from a list composed of polyamide, polyester, and blends thereof.

In one embodiment, in the knitted fabric of the sleeve of the present invention the second yarn also includes one or more active ingredients distributed throughout the second yarn selected from a list composed of witch hazel extracts, horse chestnut extracts, aloe vera extracts, pine extracts, menthol extracts, caffeine, natural antioxidants, retinol, oleic acid, fatty acids, bioactive polymers, microcapsules, phase change materials, silver particles, copper particles, and blends thereof.

In one embodiment of the sleeve of the present invention, the first area of the sleeve presents 15-20 courses, the second area has 30-40 courses and the third area has 100-200 courses.

In one embodiment of the sleeve of the present invention, the compression of the first area is 21 to 34 mmHg, particularly 23 to 32 mmHg.

In one embodiment of the sleeve of the present invention, the limb is an upper limb and the gradual reduction in compression is made in the wrist-armpit direction.

In one embodiment of the sleeve of the present invention, the sleeve comprises a first area corresponding to the palm-wrist, a second area corresponding to the elbow and a third area corresponding to the armpit.

In one embodiment of the sleeve of the present invention, the limb is a lower limb and the gradual reduction in compression is made in the ankle-groin direction.

Another aspect of the present invention comprises the circular knitted sleeve of the present invention and a fastening to avoid movement of device in relation to the wearer.

In one embodiment of the device of the present invention, the knit of the sleeve is a jersey knitted fabric between the third area and the area for fastening the device.

In one embodiment of the device of the present invention, the device also comprises an adherent material on the upper end of the sleeve to fasten the device to the wearer's limb.

In one embodiment of the device of the present invention, the adherent material is a silicone-based polymeric material selected from a list composed of polyurethane and its derivatives, polymethyl methacrylate and its derivatives, and blends thereof.

In one embodiment of the device of the present invention, it additionally comprises a second circular knitted fabric sleeve described and comprises a joining element to interconnect the two sleeves, specifically an extension of each of the pieces of knitted fabric of the sleeves.

In one embodiment of the device of the present invention, the fastening or joining element comprises a material selected from a list composed of cotton, polyester, polyamides in simple plain or satin weaves, and combinations thereof.

In one embodiment of the device of the present invention, the fastening is to attach the device to a piece of the wearer's clothing.

In one embodiment of the device of the present invention, the fastening comprises hooks, clips, straps or combinations thereof.

In one embodiment of the device of the present invention, the fastening includes a different variation in compression in the knitted fabric of each of the two sleeves in order to apply different levels of compression to each limb.

This system of reflux/graduation of compression is achieved by varying the loop length gradually in the ground structure, 1x1 rib or similar knitted structure, having in mind the changes in dimensions of the upper limbs, in combination with elastomeric materials.

This system is similar to the venous reflux mechanism in lower limbs, and the difference resides in the fact that the lymphatic system is more diffuse and is stimulated by any movement in any part of the body.

The level of compression applied to the lymphoedema depends on its condition, however, health professionals or doctors shall instruct the wearer on the range of compression that can be applied according to the classes in Table 1, i.e., mild, moderate or strong and this corresponds to the compression range that the sleeves shall have to apply to the upper limb. The sleeves developed are classified in accordance with that terminology, bearing in mind a span of perimeters of the arms in which it can be used, in order to ensure that the compression that it will apply to the lymphoedema is within the corresponding range, even if it changes slightly throughout the day.

The choice of materials relies on man-made synthetic fibres fibres and yarns, such as polyamides and polyesters, which provide low friction and have moisture management and temperature regulation properties and favour skin hydration. Emphasis is given to microfibres, hollow fibres or fibres with grooves on the surface, similar to Outlast^{R}, Coolmax^{R}, Becool^{R}, CuTec^{R}, Novarel^{R} and other fibres in the form of textured yarns in a range of linear mass densities between 44 and 120 dtex, active substances, such as silver, copper, bioactive polymers, microcapsules, phase change materials (PCMs), menthol, witch hazel, pine, horse chestnut and aloe vera extracts, caffeine, natural antioxidants, retinol, oleic acid, fatty acids or similar materials that reduce friction against the surface of the skin and hydrate the skin, similarly reducing the probability of creating skin lesions through abrasion. Furthermore, fibres and yarns that have a greater porosity favour breathability/permeability of the structures, thereby avoiding an increase in transcutaneous temperature through contact between the skin and the sleeve. The choice of elastomeric materials, in a range of linear mass densities between 200 and 300 dtex and double covered with man-made synthetic fibres, as mentioned previously, in a range of linear mass densities between 44 and 120 dtex, help control the compression/decompression of the tubular device, as well as reducing the devices' deformation capacity. These materials, which have elasticity and unparalleled recovery properties, bring together comfort, form retention, durability and freedom of movement. This performance is achieved thanks to the exclusive characteristics of the elastomeric yarn, which can be stretched up to seven times its initial length and return to its initial position once the strain has been removed.

### Process

The device is produced in small-diameter (3"-11") double cylinder knitting machines (needles in both cylinders), a technology which is linked to the production of socks and is associated with seamless knitting technology at smaller scales. Seamless technology enables garments to be manufactured with very few or even no seams or need for cutting. Both technologies have numerous advantages, specifically: flexibility in the ability to select needles; greater variety of knitted structures; ease in making changes to the design; they enable the development of several types of tubular forms in knitted fabrics; minimisation or even elimination of intensive cutting and sewing operations; economic in terms of costs and production time; greater productivity, since they offer maximum manufacturing efficiency thanks to the possibility of achieving high speeds; possibility to control loop size; reduced tension applied to the yarn; variety of types of fibres; absence of bulky, irritating seams; they provide lightness, softness, since there are no seams; more stable product quality; reduction in defects and damage due to the reduced number of operations (cutting and sewing); they provide a more attractive appearance, greater comfort and fit the body.

Knitting technology using small-dimension double cylinder machines is used to produce items suitable to the specific needs of each wearer. Compression is achieved by using elastane fibres and two different needle systems (upper and lower cylinder) as well as controlling the yarn input tension as the knitted fabric structure is produced.

The therapeutic device is knitted in a single tube of knitted fabric, of varying diameter, in accordance with the anatomical requirements of the arms, with or without lymphoedema, of the target audience. Depending on the size of the lymphoedema, this technology may not be suitable for dimensional or anatomical adaptation of the sleeve design, as in the case of treatment for final stage or class III lymphoedema, in which anatomical deformations are random. However, this device can be presented in three different types of design, produced by alterations in the finishing process after the compression tube has been produced. The format should favour freedom for normal body movements and ensure that the device is easy to put on and take off. The sleeves should be available to assist the patient in the best way possible during the recovery and treatment process. Finally, several sizes should also be available for the different stages of recovery, as well as several forms of fastening to clothing, as well as sleeves with different textures and colours.

Below, the manufacturing process is described for the therapeutic sleeve with different ways of fastening it to clothing.

The sleeve is based on a tube, in a 1x1 rib knitted fabric or similar, in which at one of the ends its diameter is considerably smaller and the compression intensity is considerably higher in comparison to the other end. The end with the smaller diameter is the wrist region, i.e., the area at the start of the sleeve, which has the greatest compression intensity. The other end relates to the armpit region and this end can be extended to the shoulder region using a jersey knitted fabric, using a sewing process, thermobonding or similar techniques in order to cover part of the shoulder. The tube should be knitted so the cut and join areas are visible.

During the knitting process, the control parameters for producing the knitted fabric should be adjusted to the compression desired in the wrist region and the graduation of the compression throughout the fabric. At the wrist, compression should be adjusted to the standardised compression class (Table 1); at the elbow, there should be a reduction in compression of 0-40%; in the armpit region there should be a reduction of 10-60% in comparison to the compression at the wrist. For Class 1 compression, the sleeve should have an intensity of 15-21 mmHg at the wrist, for Class 2, it should have an intensity of 23-32 mmHg at the wrist and for Class 3 it should have an intensity of 34-46 mmHg at the wrist. The structure to be designed should have a combination of rib knitted fabric throughout the limb that has lymphoedema (from the hand to the armpit region) and jersey knitted fabric in the extension areas for fastening the sleeve, and the rib knitted fabric is responsible for the compression and the jersey knitted fabric is responsible for the adaptability and fastening of the structure to the wearer's body. Adjusting the yarn input tension of the elastane double covered with any other fibrous material contributes to controlling the compression and, as a result, decompression along the sleeve, while the base yarn is knitted in accordance with the rules currently in use for the knitting of weft knitted fabrics. The elastane yarn, due to the use of double cylinder knitting technology, is located between the different loops of the body yarn which has active ingredients, without forming any type of loop. This elastane yarn is responsible for elasticity and therefore compression in the sleeve.

The sleeves without a fastening system finish at the end with the larger diameter with a backstitch seam in order to be completed in the sewing process with stitches known as overedge stitches. These stitches are made on overlockers, where the edge is trimmed, thereby ensuring a uniform width of the seam. The most important characteristics of this stitch are: difficult to undo, elasticity, strength and closes the garment.

During knitting of the device, the sleeve shifts from a rib knitted fabric to a jersey knitted fabric in the same tube of knitted fabric in the armpit region. Furthermore, the jersey knitted fabric does not contain elastane and is trimmed and finished in the sewing process in order to be adapted to the anatomy of a shoulder, favouring functional freedom of the upper limb. After this functional adaptation, the sleeve may have a small surface with silicone-based materials at the upper end (the end of the structure made of trimmed jersey knitted fabric), at the shoulder, measuring approximately 1 cm, to create better adherence to the surface of the skin and so as not to slide during movement. On the outer face of this end, a strap in narrow fabric is placed, together with a clip type closing system to embellish the garment and make it possible to attach it to the bra or piece of common clothing or even another sleeve

(with or without compression) using an elastic or plastic strap currently used in the bra industry. These straps pass over wearers' back region so as not to interfere with the clothing that they are wearing and so as not to be detected by others. Another way of fastening the sleeve is to join two sleeves in the sewing process so that they work like a small jacket or "bolero", where once again the fastening passes over the back.

All the sleeves manufactured undergo a heat setting process, followed by a dyeing process to introduce colour to the substrate and finally receive a silicone-based chemical finish.

In one embodiment of the present invention, the seamless knitted fabric therapeutic device with graduated compression for the treatment of lymphoedema in the upper limbs, produced by a small-diameter double cylinder knitting process, comprises a sleeve comprising fastening elements and elastomeric fibres double covered with integrated active ingredients, comprising:
- a decrease in compression of 0-40% from the palm of the hand and wrist region (5) to the elbow region (4);
- a decrease in compression of 10-60% of the amount at the elbow to the armpit region (3);
- compression in the armpit region to the shoulder lower at the armpit region.

In one embodiment, the device is characterised by compression of the palm of the hand and wrist varying between 15 and 46 mmHg.

In one embodiment, the device comprises elements for fastening the device that can be chosen from:
- silicone-based polymeric bases or similar, incorporated in the inner part of the shoulder region for fastening to the body;
- hooks, clips, straps or velcro attached to the sleeve with the possibility of fastening to another piece of clothing
- elastic or inelastic moveable or fixed straps that join two sleeves over the cervical region.

In one embodiment, the device comprises active ingredients incorporated in the fibre that consist of active substances, such as silver, copper, bioactive polymers, microcapsules, PCMs, menthol, witch hazel, pine, horse chestnut and aloe vera extracts, caffeine, natural antioxidants, retinol, oleic acid, fatty acids or similar materials.

In one embodiment, the device comprises a yarn with active properties that is on the technical face, although it can be altered to the technical back.

In one embodiment, the entire surface of the device that is in contact with the lymphoedema (3,4,5) comprises fibres that are in contact with the skin and that comprise the active ingredients that stimulate the hydration and cell regeneration of the skin and improve transcutaneous oxygenation; in the cervical region (11,12,13), the device comprises fibres with moisture management properties that provide greater breathability, such as hollow fibres or fibres that have incorporated active particles; in the shoulder region (2), it should have bioactive properties, such as particles of silver, copper or materials with the same performance; in the cervical region (10,11,12,13), it should have active properties that favour temperature regulation and moisture management.

In one embodiment, the device can be applied to prevent and/or treat lymphoedema in the upper limbs.

### Brief description of the figures

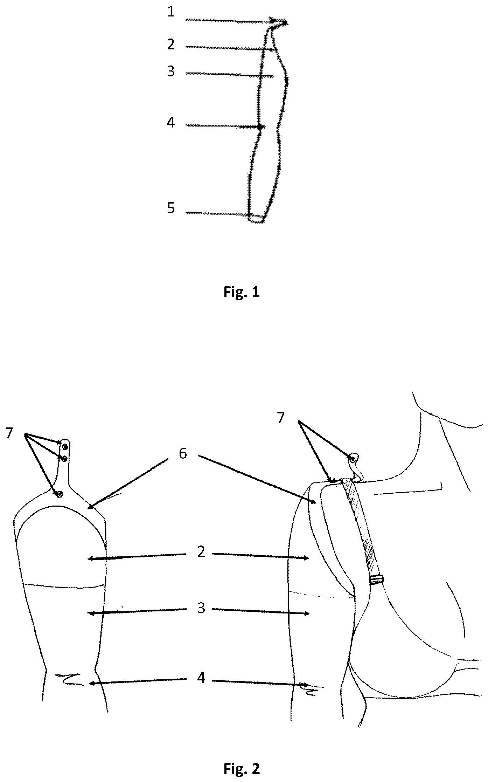

**Figure 1:** Figure 1 shows the therapeutic sleeve for upper limbs, in which the three main regions where different levels of compression should be applied are shown, in which (1,2) corresponds to the shoulder region, (3) the armpit region, (4) the elbow region and (5) the region at the start of the wrist. This sleeve can be extended to the thumb, with a lateral opening for the introduction thereof, in order to compress the back of the hand. The active ingredients should be distributed along the affected arm (3,4,5).
**Figure 2:** Figure 2 shows the regions that should be included in systems to improve the fastening of the sleeve, in which (4) is the region of the elbow with intermediate compression, (3) is the armpit region, the area with the least compression, (2) corresponds to a new knitted fabric with less elasticity to adapt to the curvature of the limb, (6) corresponds to the inner region made of material with greater adherence to the skin, such as silicone-based polymers, polyurethanes, polymethyl methacrylate and its derivatives, on the outer face the structure is strengthened with a strap to improve its appearance and facilitate the incorporation of several fastening systems and the strap can be composed of cotton, polyester, polyamides, and blends thereof, in simple plain or satin weaves and their derivatives, (7) corresponds to a system of clip fastening.

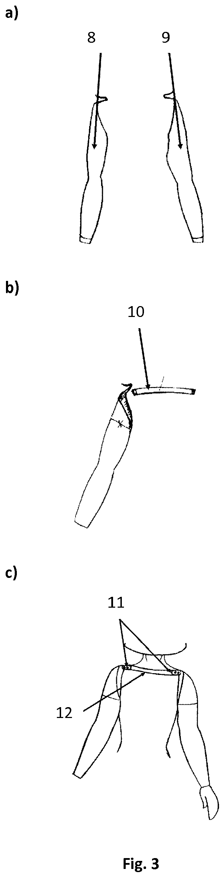

**Figure 3:** Figure 3 shows two types of sleeves that can be fastened to one another, (8) corresponds to a graduated compression sleeve and (9) corresponds to another sleeve with or without compression that is different from the one shown in (8), (10,12) corresponds to a system of elastic or inelastic straps that can be attached to the sleeves in order to join them, (11) corresponds to the system of attaching sleeves to the straps shown in (10,12).

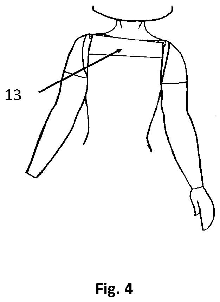

**Figure 4:** Figure 4 shows two types of sleeves that can be fastened to one another in the sewing process, (13) represents the knitted fabric that joins the two sleeves, as well as the incorporation of fibres with moisture management and temperature regulation properties.

### Detailed description

The present invention relates to a therapeutic sleeve made of knitted fabric with graduated compression that has multifunctional characteristics and that favours conditions for preventing the appearance or worsening of lymphoedema in limbs, avoiding swelling in limbs, and increasing the wearer's thermophysiological, ergonomic and psychological comfort during use.

This sleeve or therapeutic device, produced by small-diameter double cylinder knitting machines, contains structural modifications that enable several levels of compression to be obtained in the same tube of knitted fabric, with greater compression in the wrist region and less compression in the shoulder region.

The variation in compression, together with the fibrous materials used and the garment's design, as well as the possibility of attaching it to a piece of clothing, help maximise the wearer's thermophysiological and psychological comfort during use.

The preferred embodiment relates to a graduated compression sleeve, also called therapeutic device for lymphoedema in the upper limbs.

This graduated compression sleeve covers the entire upper limb and can extend from the thumb to the upper region of the shoulder, with different levels of compression. The wrist region to the armpit region (5,4,3) has different levels of compression, using the same type of materials and structures. The entire graduated compression structure structure is composed of two yarns. One of the yarns is responsible for the compression of the knitted fabric structure and is located within the knitted fabric structure and does not come into contact with the skin. This yarn is continuous filament elastomeric-based, double covered with natural or man-made fibrous materials with functional properties for temperature regulation and moisture management in order to provide greater comfort to the substrate. The other yarn is used throughout the ground structure of the sleeve and can be composed of active materials to encourage skin hydration, cell regeneration and the activation of transcutaneous oxygenation, as well as favouring breathability, moisture management and temperature regulation. The yarn used in the ground structure is also in contact with the skin, and can be composed of fibres that include active ingredients, such as menthol, witch hazel extracts, pine, horse chestnut extracts, aloe vera extracts and silver particles, copper particles and other active materials (5,4,3). The structure used to manufacture the sleeve is rib knit and its derivatives, compression is applied by the combination of the selected structure, by the composition of the fibrous materials, by the yarn input tensions (0.1-0.5 g/tex) and by the loop lengths (lu 0.002-0.08 mm) used, which is greater in the wrist region (5), with values between 15 and 46 mmHg in the range of courses in the wrist-armpit direction, as already mentioned, in order to guarantee a reduction in compression in that direction, bearing in mind the requirements imposed for this type of device. This variation every 15-20 courses, at the wrist, at an increased interval of 30-40 courses at the elbow and 100-200 courses between the elbow and the armpit. These intervals depend on the length of the sleeve in order to ensure that the compression applied between the wrist and the elbow and the elbow and the armpit are complied with, in accordance with point [0005], this increase in loop depends on the intervals but varies within a [5-20%] range each time.The structure for manufacturing the extension to the compression sleeve to the shoulder (2) uses a jersey knit or one of its derivatives and the two yarns used are knitted by plating, in which the yarn that is in contact with the skin should encourage thermophysiological comfort and, subsequently, psychological comfort, with attractive or discrete colours and design, depending on its use. This structure should be relatively inelastic in order to stabilise the sleeve on the arm, and should avoid shifting on the arm and, at the same time, provide freedom of movement for the limb.

In one embodiment, the systems for fastening the sleeves can be one of three types, including a clip type closing system (7), hooks or other similar systems that can be fastened to a bra or to the inside of a piece of clothing, such as a shirt. The second fastening system uses narrow bands of elastic or inelastic fabric or even polymeric straps (10,12) which join two sleeves, at least one of which has graduated compression for treating lymphoedema. These systems have different aesthetic characteristics and can have colours, patterns and can be transparent or semi-transparent. These materials can have characteristics that favour the reduction of friction between the material and the skin. The third fastening system consists of joining two sleeves, at least one of which has graduated compression for treating lymphoedema, and these sleeves have an extension in the knitted fabric (2) to later be joined in the sewing process. In this case, the structure's knitted fabric (2) should have moisture management and temperature regulation properties, through the use of fibres with these functions or by introducing active substances through a chemical process.

This description is not, naturally, restricted in any way to the embodiments described in this document and a person with ordinary skill in the art could envisage many possibilities for modification thereof and substitutions of technical characteristics by other equivalent characteristics, depending on the requirements of each situation, as established in the attached claims.

The preferred embodiments described above can be used in combination with one another. The following claims further define the preferred embodiments.

## Claims

1. Seamless circular knitted fabric sleeve for treating lymphoedema in one of a wearer's limbs, obtainable by small-diameter double cylinder knitting,
wherein the sleeve comprises a plurality of areas with different compression levels in order to gradually reduce the compression applied to the wearer's limb in the distal to proximal direction,
in which the loop lengths of the knitted fabric and the yarn knitting input tension are distributed along the sleeve such that compression varies from 15-46 mmHg in a first area of the sleeve, decreasing 0-40% until a second area of the sleeve; and such that compression varies from the second area of the sleeve, decreasing 10-60% until a third area of the sleeve.

2. Sleeve according to the preceding claims in which the yarn tension varies between 0.1-0.5 g/tex and the loop length varies between 0.002-0.08 mm, wherein each loop length of an area of the sleeve varies 5-20% in relation to each loop length in the preceding area.

3. Sleeve according to the preceding claims wherein the knitted fabric comprises at least two yarns: a first yarn and a second yarn.

4. Sleeve according to the preceding claim wherein the first yarn is elastomeric with a linear mass density 200-300 dtex, double covered with man-made synthetic fibres with a linear mass between 44-120 dtex and in which the man-made synthetic fibres are selected from a list composed of polyamides, polyesters, or blends thereof.

5. Sleeve according to claims 3 or 4 wherein the second yarn is made of a material selected from a list composed of polyamide, polyester, or blends thereof.

6. Sleeve according to the preceding claim wherein the second yarn also comprises one or more active ingredients distributed throughout the second yarn selected from a list composed of witch hazel extracts, horse chestnut extracts, aloe vera extracts, pine extracts, menthol extracts, caffeine, natural antioxidants, retinol, oleic acid, fatty acids, bioactive polymers, microcapsules, phase change materials, silver particles, copper particles, or blends thereof.

7. Sleeve according to the preceding claims wherein the first area of the sleeve has 15-20 courses, the second area has 30-40 courses and the third area has 100-200 courses.

8. Sleeve according to the preceding claims wherein the compression of the first area is of 21 to 34 mmHg, particularly 23 to 32 mmHg.

9. Sleeve according to the preceding claims wherein the limb is an upper limb, wherein the gradual reduction in compression is made in the wrist-armpit direction.

10. Sleeve according to the preceding claim wherein:
the first area corresponds to the palm-wrist,
the second area corresponds to the elbow,
the third area corresponds to the armpit.

11. Sleeve according to the preceding claims wherein the limb is a lower limb, wherein the gradual reduction of compression is made in the ankle-groin direction.

12. Device that comprises a circular knitted fabric sleeve described in any one of the preceding claims and a fastening to avoid the device moving relative to the wearer.

13. Device according to the preceding claim wherein the knit is a jersey structure between the third area and the fastening area of the device.

14. Device according to claims 12-13 that further comprises an adherent material on the upper end of said sleeve to fasten the device to the wearer's limb.

15. Device according to the preceding claim wherein the adherent material is a silicone-based polymeric material selected from a list composed of polyurethane and its derivatives, polymethyl methacrylate and its derivatives, or blends thereof.

16. Device according to claims 12-15 that additionally comprises a second circular knitted sleeve described in any of claims 1-11, and that comprises a joining element to interconnect the two sleeves, in particular an extension of each of the pieces of knitted fabric of the sleeves.

17. Device according to claims 12-16 wherein the fastening or joining element comprises a material selected from a list composed of cotton, polyester, polyamides, in simple plain or satin weaves, or combinations thereof.

18. Device according to claims 12-17 wherein the fastening is to attach the device to a piece of the wearer's clothing.

19. Device according to claims 12-18 in which the fastening comprises hooks, clips, straps or combinations thereof.

20. Device according to claims 12-19 wherein the variation in compression of the knitted fabric of each of the sleeves is different.
